(19) Europäisches Patentamt
European Patent Office
Office européen des brevets

(11) Veröffentlichungsnummer: **0 187 286**
**A1**

(12) # EUROPÄISCHE PATENTANMELDUNG

(21) Anmeldenummer: **85115644.8**

(22) Anmeldetag: **09.12.85**

(51) Int. Cl.[4]: **A 61 K 39/39**
**A 61 K 39/00**

(30) Priorität: **20.12.84 DE 3446515**

(43) Veröffentlichungstag der Anmeldung:
**16.07.86 Patentblatt 86/29**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Bernhardt, Dieter, Dr.**
**Goldbergstrasse 18a**
**D-3553 Cölbe(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Öladjuvierte Vaccine und Verfahren zu ihrer Herstellung.**

(57) Es wird ein Verfahren zur Adjuvierung einer Vaccine beschrieben, in welchem einer Antigenlösung ein Öl, ein Sorbitan-oleat, eine Verbindung der Formel I

$$HO(CH_2O)_a(\underset{\displaystyle CH_3}{\underset{|}{C}}HCH_2O)_b(CH_2CH_2O)_cH \qquad I$$

mit einem Molekulargewicht von 4000 bis 5500, wobei a und c statistisch gleich sind und b eine solche Zahl bedeutet, daß das Gewichtsverhältnis des Oxyethylenanteils zu dem Oxypropylenanteil 7:3 bis 9:1 ist, gegebenenfalls ein Polyoxyethylen-sorbitan-monooleat, gegebenenfalls Glycerin und gegebenenfalls ein mineralisches Adjuvans, Saponin und/oder ein als adjuvierend bekannter Pflanzenextrakt zugesetzt wird, sowie eine auf diese Weise adjuvierte Vaccine.

EP 0 187 286 A1

0187286

Öladjuvierte Vaccine und Verfahren zu ihrer Herstellung

Die Erfindung betrifft eine öladjuvierte Vaccine sowie ein Verfahren zu ihrer Herstellung.

Viele Antigene sind so wenig immunogen, daß sie bei einer einmaligen Injektion in ein Tier keine meßbare oder nur eine geringe Immunantwort auslösen, so daß sie nicht ohne weiteres als Impfstoff verwendet werden können. Die antigene Wirksamkeit kann verstärkt werden, indem diese Antigene mit einem Immunstimulator oder Adjuvans zusammen angewendet werden. Die meisten inaktivierten Virus-und Bakterienvaccinen enthalten aus diesem Grund Adjuvantien.

Es ist bekannt, wässrige Antigenlösungen durch Emulgieren mit Mineral- oder Pflanzenölen zu adjuvieren.

Solche Emulsionen, besonders Mineralölemulsionen, werden jedoch lokal schlecht vertragen. Deshalb werden solche Emulsionen oft intramuskulär angewandt, um eine bessere Verträglichkeit vorzutäuschen. Da sich jedoch an der Injektionsstelle ein steriler Abszeß entwickelt, wird dieses Vorgehen aus fleischbeschaurechtlichen Gründen bei Nutztieren zunehmend abgelehnt. Weiterhin sind Ölemulsionsvaccinen verhältnismäßig viskos, wodurch der Umgang mit ihnen erschwert ist. Der Ölgehalt solcher Vaccinen liegt üblicherweise zwischen 30 und 50 %. Eine Verringerung des Ölgehaltes ist nicht möglich, da die Vaccinen dann pastenartig würden und nicht mehr injiziert werden können. Weiterhin benötigt man für jedes Öl einen für dieses Öl geeigneten spezifischen Emulgator.

0187286

Es wurde nun überraschenderweise gefunden, daß eine Kombination eines Öls mit bestimmten Emulgatoren zur Adjuvierung von Vaccinen geeignet ist, wobei die gerade beschriebenen Nachteile nicht beobachtet werden.

Gegenstand der Erfindung ist daher eine Vaccine enthaltend eine wässrige Antigenlösung und eine Adjuvans-Emulsion gekennzeichnet durch einen Gehalt an einem Öl, einem Sorbitan-Oleat, einer Verbindung der Formel I

$$HO(CH_2O)_a(\underset{CH_3}{CH}CH_2O)_b(CH_2CH_2O)_cH \qquad I$$

mit einem Molekulargewicht von 4000 bis 5500, wobei a und c statistisch gleich sind und b eine solche Zahl bedeutet, daß das Gewichtsverhältnis des Oxyethylenanteils zu dem Oxypropylenanteil 7:3 bis 9:1 ist, gegebenenfalls einem Polyoxyethylen-sorbitan-monooleat und gegebenenfalls Glycerin.

Das Öl kann ein Mineral- oder ein Pflanzenöl sein. Die adjuvierenden Bestandteile werden vorteilhafterweise vor der Zugabe zu der wässrigen Antigenlösung miteinander vermischt und mit einer wässrigen Phase emulgiert, bevor sie der Antigenlösung zugesetzt werden.

Das Sorbitan-Oleat ist ein Mono- oder Trioleat, vorzugsweise ein Monooleat.

Bevorzugt enthält eine solche Vaccine eine Adjuvans-Emulsion, die 5-20 ml, vorzugsweise 8-12 ml, eines Öls, vorzugsweise Mineralöl, 0,5-10 ml, vorzugsweise 1-5 ml, Sorbitan-Oleat und 0,5-10 g, vorzugsweise 1-4 g, einer Verbindung der Formel I auf 100 ml enthält.

Gegebenenfalls enthält diese weiterhin 0,5-10 g, vorzugsweise 0,5-4 g, eines Polyoxyethylen-sobitan-monooleats und gegebenenfalls 0,5-10, vorzugsweise 0,5-3 ml, Glycerin auf 100 ml.

Das Volumenverhältnis von wässriger Antigenlösung zu Adjuvans-Emulsion kann 0,5 bis 2, vorzugsweise 0,8 bis 1,2 betragen.

Als Mineralöl werden Bayol® F oder Marcol® 52 bevorzugt. Es können aber auch Paraffinöl (rein weiß Merck), Drakeol® oder Miglyol® verwendet werden.

Das Antigen kann vor allem ein Virus-, Bakterien-, Zell- oder Proteinantigen sein.

Als weitere immunstimulatorische Substanzen können übliche Adjuvantien wie Saponin und weiterhin Pflanzenextrakte, Aluminiumhydroxid, Aluminiumphosphat, Kaolin, Bentonit, Kieselgur oder Kieselsäuren zugesetzt werden.

Die erfindungsgemäße Adjuvierung bewirkt eine höhere Immunstimulation verglichen mit den Verfahren des Standes der Technik.

Die dazu nötige Menge an Adjuvans ist geringer als bei bekannten Verfahren. Es werden vorzugsweise solche Mengen verwendet, daß in der Vaccine bis zu 15 ml Adjuvans in 100 ml fertiger Vaccine enthalten sind. Es können aber auch größere Mengen an Adjuvans zugegeben werden.

In der Regel genügt einfaches Schütteln einer wässrigen Antigenlösung mit einem erfindungsgemäßen Adjuvansgemisch, um eine stabile Emulsion zu erhalten.

Die Vaccinen können übliche Hilfs- oder Zusatzstoffe enthalten.

Die folgenden Beispiel sollen die Erfindung erläutern.

Beispiel 1

Herstellung einer adjuvierten Vaccine

0,5 ml Paraffinöl; Siedebereich 190-240°C (Bayol® F),
0,1 ml Sorbitan-trioleat; MW etwa 1050,
0,1 ml Polyoxyethylen-sorbitan-trioleat, MW etwa 1300,
0,2 ml einer Lösung von 40 g der Verbindung der Formel I, MW 4750, Gewichtsverhältnis Oxyethylen/Oxypropylen-Anteil 8:2, in 100 ml Wasser und
0,4 ml Glycerin wurden gemischt und zu der entstandenen Emulsion 5 ml Antigen (Virus diarrhoe/Mucosal disease-Antigen) in Wasser und 3,7 ml phosphatgepufferte Kochsalzlösung (PBS) langsam gegeben und vermischt.

Die entstandene Wasser in Öl-Emulsion blieb nach der Mischung stabil.

Wenn anstelle von Bayol® F Drakeol®, Marcol® 52, Paraffinöl (rein weiß Merck) oder Miglyol® verwendet wurde, entstanden ebenfalls stabile Emulsionen.

Wirksamkeit und Verträglichkeit

Die Vaccine (je 3 ml) wurde Kaninchen subkutan verabreicht. Als Vergleich diente eine Vaccine, die 5 ml Antigenlösung und 5 ml Complete Freund's Adjuvans (FCA) enthielt.

Während diese 3 Wochen nach der Applikation an der

Injektionsstelle etwa 30 Millimeter große Verhärtungen zeigte, waren die Verhärtungen bei der erfindungsgemäß mit Bayol F adjuvierten Vaccine lediglich erbsengroß.

Im Neutralisationstest wurden die Antikörper gegen VD/MD Virus bestimmt. Der Titer der Seren von Kaninchen, die mit FCA enthaltender VD/MD-Vaccine geimpft worden waren, war 1:140; erfindungsgemäß mit Bayol F 1:500.

Beispiel 2

In Beispiel 1 wurde anstelle von VD/MD-Antigen MKS-Antigen verwendet. Die Vaccine (je 0,4 ml) wurde Mäusen appliziert. 3 Wochen danach wurden die Tiere mit MKS-Virus belastet. Dabei wies die Vaccine eine 50 %-schützende Dosis ($PD_{50}$) von 4,3 auf.
Vergleich: FCA-haltige MKS-Vaccine      0,2 $PD_{50}$.

Beispiel 3

| | | |
|---|---|---|
| Vaccine A | 0,5 ml | Paraffinöl Marcol® 52 |
| | 0,1 ml | Sorbitan-trioleat, MW ca. 1050, |
| | 0,1 ml | Polyoxyethylen-sorbitan-trioleat, MW etwa 1300, |
| | 0,2 ml | einer Lösung von 40 g der Verbindung der Formel I, MW 4750, Gewichtsverhältnis Oxyethylen/Oxypropylen-Anteil 8:2, in 100 ml Wasser, |
| | 0,4 ml | Glycerin und |
| | 0,5 ml | Saponin, 1 %ig in Wasser, |

wurden gemischt. Danach wurden 5,0 MKS-Antigen und 3,2 ml PBS zugemischt.

| | | |
|---|---|---|
| Vaccine B | 5,0 ml | FCA und |
| | 5,0 ml | MKS-Antigen. |

Vaccine C   Wie Vaccine A aber ohne Saponin.

Mit diesen 3 Vaccinen wurden Mäuse immunisiert und 3 Wochen nach Impfung mit infektiösem MKS-Virus belastet. Die Resultate waren wie folgt:

| | |
|---|---|
| Vaccine A | 3,7 $PD_{50}$/0,4 ml |
| Vaccine B | 0,2 $PD_{50}$/0,4 ml |
| Vaccine C | 0,8 $PD_{50}$/0,4 ml. |

Dieses Beispiel zeigt, daß durch Zugabe von Saponin die immunstimulatorische Wirkung der Ölvaccinen noch erheblich gesteigert werden kann.

Beispiel 4

| | |
|---|---|
| 0,5 ml | Paraffinöl; Siedebereich 190-240°C, |
| 0,1 ml | Sorbitan-trioleat; MW etwa 1050, |
| 0,1 ml | Polyoxyethylen-sorbitan-trioleat, MW etwa 1300, |
| 0,2 ml | einer Lösung von 40 g der Verbindung der Formel I, MW 4750, Gewichtsverhältnis Oxyethylen/Oxypropylen-Anteil 8:2, in 100 ml Wasser, |
| 0,4 ml | Glycerin und |
| 8,7 ml | PBS |

wurden zu einer Emulsion gemischt und Mäusen je 0,2 ml s.c. appliziert. 3 Tage später wurden diese Mäuse sowie unbehandelte Mäuse mit einer tödlichen Dosis MKS-Virus oder V. cholerae belastet. Während alle unbehandelten Tiere starben, überlebten von denen, welche die Ölemulsion erhalten hatten, bei der Gabe von

| | |
|---|---|
| V. cholerae | 50 % und von |
| MKS-Virus | 60 %. |

Dies zeigt, daß mit diesen Präparationen eine Immunstimulation erreicht wird, die nicht auf einer Bildung von Antikörpern beruht, die sich aber in einer erhöhten Resistenz gegenüber tödlichen Virus- und Bakterieninfektionen manifestiert (Paraimmunität).

## Beispiel 5

### Herstellung einer adjuvierten Vaccine

0,5 ml Paraffinöl; Siedebereich 190-240°C (Bayol® F),
0,1 ml Sorbitan-monooleat; MW etwa 1050,
0,1 ml Polyoxyethylen-sorbitan-monooleat, MW etwa 1300,
0,2 ml einer Lösung von 40 g der Verbindung der Formel I, MW 4750, Gewichtsverhältnis Oxyethylen/Oxypropylen-Anteil 8:2, in 100 ml Wasser

wurden gemischt und zu der entstandenen Emulsion 5 ml Antigen (Virus diarrhoe/Mucosal disease-Antigen) in Wasser und 4,1 ml phosphatgepufferte Kochsalzlösung (PBS) zugegeben und vermischt.

Die entstandene Wasser in Öl-Emulsion blieb nach Zentrifugation stabil.

Wenn anstelle von Bayol® F, Drakeol®, Marcol® 52, Paraffinöl (rein weiß Merck) oder Miglyol® verwendet wurde, entstanden ebenfalls stabile Emulsionen.

### Wirksamkeit und Verträglichkeit

Die Vaccine (je 3 ml) wurde Kaninchen subkutan verabreicht. Als Vergleich diente eine Vaccine, die 5 ml Antigenlösung und 5 ml Complete Freund's Adjuvans (FCA) enthielt.

Während diese 3 Wochen nach der Applikation an der Injektionsstelle etwa 30 Millimeter große Verhärtungen zeigte, waren die Verhärtungen bei der erfindungsgemäß mit Bayol® F adjuvierten Vaccine lediglich erbsengroß.

Im Neutralisationstest wurden die Antikörper gegen VD/MD Virus bestimmt. Der Titer der Seren von Kaninchen, die mit FCA enthaltender VD/MD-Vaccine geimpft worden waren, war 1:140; erfindungsgemäß mit Bayol® F 1:500.

## Beispiel 6

In Beispiel 5 wurde anstelle von VD/MD-Antigen MKS-Antigen verwendet. Die Vaccine (je 0,4 ml) wurde Mäusen appliziert. 3 Wochen danach wurden die Tiere mit MKS-Virus belastet. Dabei wies die Vaccine eine 50 %-schützende Dosis ($PD_{50}$) von 4,3 auf.
Vergleich: FCA-haltige MKS-Vaccine 0,2 $PD_{50}$.

## Beispiel 7

| | | |
|---|---|---|
| Vaccine A | 0,5 ml | Paraffinöl Marcol® 52 |
| | 0,1 ml | Sorbitan-monooleat, MW ca. 1050, |
| | 0,1 ml | Polyoxyethylen-sorbitan-monooleat, MW etwa 1300, |
| | 0,2 ml | einer Lösung von 40 g der Verbindung der Formel I, MW 4750, Gewichtsverhältnis Oxyethylen/Oxypropylen-Anteil 8:2, in 100 ml Wasser und |
| | 0,5 ml | Saponin, 1 %ig in Wasser, |

wurden gemischt. Danach wurden 5,0 ml MKS-Antigen und 3,6 ml PBS zugemischt.

| | | |
|---|---|---|
| Vaccine B | 5,0 ml | FCA und |
| | 5,0 ml | MKS-Antigen. |

Vaccine C Wie Vaccine A aber ohne Saponin.

Mit diesen 3 Vaccinen wurden Mäuse immunisiert und 3 Wochen nach Impfung mit infektiösem MKS-Virus belastet. Die Resultate waren wie folgt:

| | |
|---|---|
| Vaccine A | 3,7 $PD_{50}$/0,4 ml |
| Vaccine B | 0,2 $PD_{50}$/0,4 ml |
| Vaccine C | 0,8 $PD_{50}$/0,4 ml. |

Dieses Beispiel zeigt, daß durch Zugabe von Saponin die immunstimulatorische Wirkung der Ölvaccinen noch erheblich gesteigert werden kann.

Beispiel 8

| | |
|---|---|
| 0,5 ml | Paraffinöl; Siedebereich 190-240°C, |
| 0,1 ml | Sorbitan-monooleat; MW etwa 1050, |
| 0,1 ml | Polyoxyethylen-sorbitan-monooleat, MW etwa 1300, |
| 0,2 ml | einer Lösung von 40 g der Verbindung der Formel I, MW 4750, Gewichtsverhältnis Oxyethylen/Oxypropylen-Anteil 8:2, in 100 ml Wasser und |
| 9,1 ml | PBS |

wurden zu einer Emulsion gemischt und Mäusen je 0,2 ml s.c. appliziert. 3 Tage später wurden diese Mäuse sowie unbehandelte Mäuse mit einer tödlichen Dosis MKS-Virus oder V. cholerae belastet. Während alle unbehandelten Tiere starben, überlebten von denen, welche die Ölemulsion erhalten hatten, bei der Gabe von

| | |
|---|---|
| V. cholerae | 50 % und von |
| MKS-Virus | 60 %. |

Dies zeigt, daß auch mit diesen Präparationen eine Immunstimulation erreicht wird, die nicht auf einer Bildung von Antikörpern beruht, die sich aber in einer erhöhten Resistenz gegenüber tödlichen Virus- und Bakterieninfektionen manifestiert (Paraimmunität).

Beispiel 9

Vaccine A 0,5 ml Paraffinöl Marcol® 52
0,025 ml Sorbitan-monooleat; MW etwa 1050
0,025 ml Polyoxyethylen-sorbitan-monooleat, MW etwa 1300
0,5 ml einer Lösung von 40 g der Verbindung der Formel I, MW 4750, Gewichtsverhältnis Oxyethylen/Oxypropylen-Anteil 8:2, in 100 ml Wasser

wurden gemischt, danach wurden 5,0 ml MKS-Antigen und 3,95 ml PBS zugemischt.

Vaccine B 0,5 ml Paraffinöl Marcol® 52
0,025 ml Sorbitan-monooleat; MW etwa 1050
0,025 ml Polyoxyethylen-sorbitan-monooleat, MW etwa 1300
0,5 ml einer Lösung von 40 g der Verbindung der Formel I, MW 4750, Gewichtsverhältnis Oxyethylen/Oxypropylen-Anteil 8:2, in 100 ml Wasser
0,4 ml eine Suspension von 20 g Kieselgur (Filtercel®) in 100 ml 0,1 mol Phosphatpuffer pH 7,3

wurden gemischt, danach wurden 5,0 ml MKS-Antigen und 3,2 ml PBS zugemischt.

Mit diesen beiden Vaccinen wurden Mäuse immunisiert und 3 Wochen nach Impfung mit infektiösem MKS-Virus belastet. Die Resultate waren wie folgt:

Vaccine A 3,2 $PD_{50}$/0,4 ml
Vaccine B 6,8 $PD_{50}$/0,4 ml

Dieses Beispiel zeigt, daß durch Zugabe von Kieselgur die immunstimulatorische Wirkung der Ölvaccine noch gesteigert werden kann.

Patentansprüche:

1. Vaccine enthaltend eine wässrige Antigenlösung und eine Adjuvans-Emulsion gekennzeichnet durch einen Gehalt an einem Öl, einem Sorbitan-oleat, einer Verbindung der Formel I

$$HO(CH_2O)_a(\underset{\displaystyle CH_3}{CH}CH_2O)_b(CH_2CH_2O)_cH \qquad I$$

mit einem Molekulargewicht von 4000 bis 5500, wobei a und c statistisch gleich sind und b eine solche Zahl bedeutet, daß das Gewichtsverhältnis des Oxyethylenanteils zu dem Oxypropylenanteil 7:3 bis 9:1 ist, gegebenenfalls einem Polyoxyethylen-sorbitan-monooleat, gegebenenfalls Glycerin und gegebenenfalls weitere Adjuvantien.

2. Vaccine nach Anspruch 1, dadurch gekennzeichnet, daß sie eine Adjuvans-Emulsion enthält, die 5-20 ml, vorzugsweise 8-12 ml, eines Öls, vorzugsweise Mineralöl, 0,5-10 ml, vorzugsweise 1-5 ml, Sorbitan-oleat und 0,5-10 g, vorzugsweise 1-4 g, einer Verbindung der Formel I auf 100 ml enthält.

3. Vaccine nach Anspruch 1, dadurch gekennzeichnet, daß sie eine Adjuvans-Emulsion enthält, die 0,5-10 g, vorzugsweise 0,5-4 g, eines Polyoxyethylen-sorbitanmonooleats und gegebenenfalls 0,5-10 ml, vorzugsweise 0,5-3 ml, Glycerin auf 100 ml enthält.

4. Vaccine nach Anspruch 1, dadurch gekennzeichnet, daß das Volumenverhältnis von wässriger Antigenlösung zu Adjuvans-Emulsion 0,05 bis 0,3, vorzugsweise 0,1 bis 0,2 beträgt.

5. Vaccine nach Anspruch 1, dadurch gekennzeichnet, daß sie weiterhin ein mineralisches Adjuvans, Saponin und/

oder einen als adjuvierend bekannten Pflanzenextrakt enthält.

6. Verfahren zur Herstellung einer Vaccine nach Anspruch 1, dadurch gekennzeichnet, daß einer Antigenlösung ein Öl, ein Sorbitan-oleat, eine Verbindung der Formel I, gegebenenfalls ein Polyoxyethylen-sorbitan-monooleat, gegebenenfalls Glycerin und gegebenenfalls ein mineralisches Adjuvans, Saponin und/oder ein als adjuvierend bekannter Pflanzenextrakt zugesetzt wird.

7. Verfahren zur Herstellung einer Vaccine nach Anspruch 1, dadurch gekennzeichnet, daß das Öl, das Sorbitan-oleat, die Verbindung der Formel I, gegebenenfalls das Polyoxyethylen-sorbitan-monooleat, gegebenenfalls das Glycerin und gegebenenfalls das mineralische Adjuvans, Saponin und/oder ein als adjuvierend bekannter Pflanzenextrakt miteinander vermischt und mit einer wässrigen Phase emulgiert werden, bevor sie der Antigenlösung zugesetzt werden.

Europäisches Patentamt

# EUROPÄISCHER RECHERCHENBERICHT

0187286

Nummer der Anmeldung

EP 85 11 5644

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X,Y | EP-A-0 121 752 (BEHRINGWERKE AG) * Seite 7, Zeile 1 - Seite 8, Zeile 6; Ansprüche 1-7 * --- | 1-7 | A 61 K 39/39<br>A 61 K 39/00 |
| Y | GB-A-1 128 325 (THE WELCOME FOUNDATION LTD.) * Seite 1, Zeile 12 - Seite 2, Zeile 67 * --- | 1-7 | |
| Y | FR-M- 4 690 (MERCK & CO., INC.) * Seite 9, Zusammenfassung * --- | 1-7 | |
| A | CHEMICAL ABSTRACTS, Band 77, Nr. 5, 31. Juli 1972, Seite 137, Nr. 30033v, Columbus, Ohio, US; R. MURRAY et al.: "Mineral oil adjuvants. Biological and chemical studies", & ANN. ALLERGY 1972, 30(3), 146-51 * Zusammenfassung * --- | 1-7 | RECHERCHIERTE SACHGEBIETE (Int. Cl.4)<br>A 61 K |
| A | EP-A-0 059 521 (CENTRAAL DIERGENEESKUNDIG INSTITUUT) * Seite 7, Zeilen 1-4; Anspruch 1 * --- | 1-7 | |
| A | GB-A-1 083 815 (THE WELLCOME FOUNDATION LTD.) * Seite 1, Zeilen 43-59 * --- -/- | 1-7 | |

Der vorliegende Recherchenbericht wurde für alle Patentansprüche erstellt

| Recherchenort | Abschlußdatum der Recherche | Prüfer |
|---|---|---|
| DEN HAAG | 27-03-1986 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

0187286

Nummer der Anmeldung

EP 85 11 5644

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-1 081 796 (THE WRIGHT-FLEMING INSTITUTE) * Seite 3, Zeilen 8-33; Ansprüche 1-5 * --- | 1-7 | |
| A | GB-A-1 035 701 (MERCK & CO., INC.) * Seite 13, Zeilen 3-37; Ansprüche 1-8 * ----- | 1-7 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 27-03-1986 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82